# EUROPÄISCHE PATENTSCHRIFT

(11) **EP 1 277 490 B1**
(45) Veröffentlichungstag und Bekanntmachung des Hinweises auf die Patenterteilung: **21.06.2006**
(21) Anmeldenummer: 01117239.2
(22) Anmeldetag: 17.07.2001
(51) Int. Cl.: A61M 39/14

(54) **Einstechdorn**
Spike connector
Connecteur à aiguille

(43) Veröffentlichungstag der Anmeldung: 22.01.2003
(73) Patentinhaber: Codan Holding GmbH, 23738 Lensahn (DE)
(72) Erfinder: Crombach, Josef J. M., 8251 VH Deventer (NL)
(74) Vertreter: Fleck, Thomas

(56) Entgegenhaltungen:
- EP-A- 0 245 119
- EP-A- 0 327 519

## Beschreibung

Die vorliegende Erfindung betrifft einen Einstechdorn.

Die EP 0 245 119 A1 offenbart einen einteiligen Einstechdorn, der für ein Verabreichungsset der Infusionstherapie zum Entnehmen von Flüssigkeit aus einem Behältnis geeignet ist, und der einen einlumigen Flüssigkeitskanal, eine abgerundete und schräg verlaufende Spitze, sowie eine Griffplatte besitzt. Dieser vorbekannte Einstechdorn ist jedoch nicht zum Beimischen von Medikamenten geeignet, da kein zusätzlicher Anschlußstutzen vorgesehen ist.

In der EP 0 327 519 A1 wird zwar schon ein Luer-(Lock) Anschluß 66 beschrieben, jedoch werden dort weder Größenangaben zum Innendurchmesser des Flüssigkeitskanals, noch zum Tot-raumvolumen im Bereich des Anschlußstutzens gemacht. Der Luer-Lock Anschluß 66 bedarf im Gebrauch eines Ventilstopfens 52 in Form eines Rückschlagventils und ist somit zweiteilig. Außerdem besitzt diese relativ komplexe Einrichtung eine zweite Verbindungsöffnung 14 ohne Luer-Lock Anschluß, so daß sehr große Totraumvolumina entstehen, je nach dem, mit welchem Zugang gearbeitet wird.

Der vorliegenden Erfindung liegt deshalb die Aufgabe zugrunde, die vorbekannten Einstechdorne derart zu verbessern, daß sie sicher, einfach und schnell ohne Verletzungs- oder Beschädigungsrisiko gehandhabt werden können, und sich für die Medikamentenmisch- und Verabreichungssets eignen.

Die vorliegende Aufgabe wird durch den im Hauptanspruch gekennzeichneten einteiligen Einstechdorn gelöst.

Die Summation der erfindungsgemäßen Merkmale ergibt in überraschenderweise eine einfache, schnelle und sichere Handhabungsweise des Einstechdorns. Insbesondere weist der einlumige Flüssigkeitskanal im Einstechdorn einen größeren besonders abgestimmten Innendurchmesser auf, wodurch ein schnellerer sicher ablaufenden Flüssigkeitstransfer für hochwirksame Medikamente erreicht und ein Schlauchanschluß mit negativer Schlauchverklebung möglich wird.

Das Versehen des erfindungsgemäßen einteiligen Einstechdorns mit einem integrierten Luer-(Lock) negativ oder zylindrischen Anschlußstutzen ermöglicht die direkte Montage/Verklebung von Anschlußstücken mit einem Absperrmechanismus oder Zuspritzmembranen am erfindungsgemäßen Einstechdorn.

Somit entfällt der Einsatz eines zusätzlichen Verbindungsstücks, wodurch die Herstellung vereinfacht und die Wirtschaftlichkeit erhöht wird. Außerdem wird die Sicherheit des erfindungsgemäßen Einstechdorns durch weniger Montageklebestellen erhöht und das Totraumvolumen, wichtig für hochwirksame Medikamente mit geringem Verabreichungsvolumen, in dem angesprochenen Bereich erheblich reduziert.

Die vorteilhafte Kontur der Griffplatte in gewölbter Form sichert schließlich wiederum die leichtere und sicherere Handhabung beim Einstechen des Dornes in das Infusionsbehältnis, insbesondere den Infusionsbeutel.

Weitere Vorteile und Merkmale gehen aus den Unteransprüchen hervor, die insbesondere in Zusammenhang mit dem Hauptanspruch von erfinderische Bedeutung sein können.

Zum besseren Verständnis der Erfindung wird letztere im Zusammenhang mit der Darstellung des Standes der Technik vierglichen und erläutert.

Es zeigt:
- Fig. 1: eine schematische Darstellung eines Misch- und Verabreichungssets nach dem Stand der Technik;
- Fig. 2: eine schematische Querschnittsansicht des in Fig. 1 gezeigten Einstechdorns in Form einer vergrößerten Detailansicht;
- Fig. 3: eine schematische Frontansicht des erfindungsgemäßen Einstechdorns;
- Fig. 4: eine schematische Seitenansicht des in Fig. 3 gezeigten erfindungsgemäßen Einstechdorns; und
- Fig. 5: eine schematische Seitenansicht des erfindungsgemäßen Einstechdorns mit aufgeschraubtem Adapter.

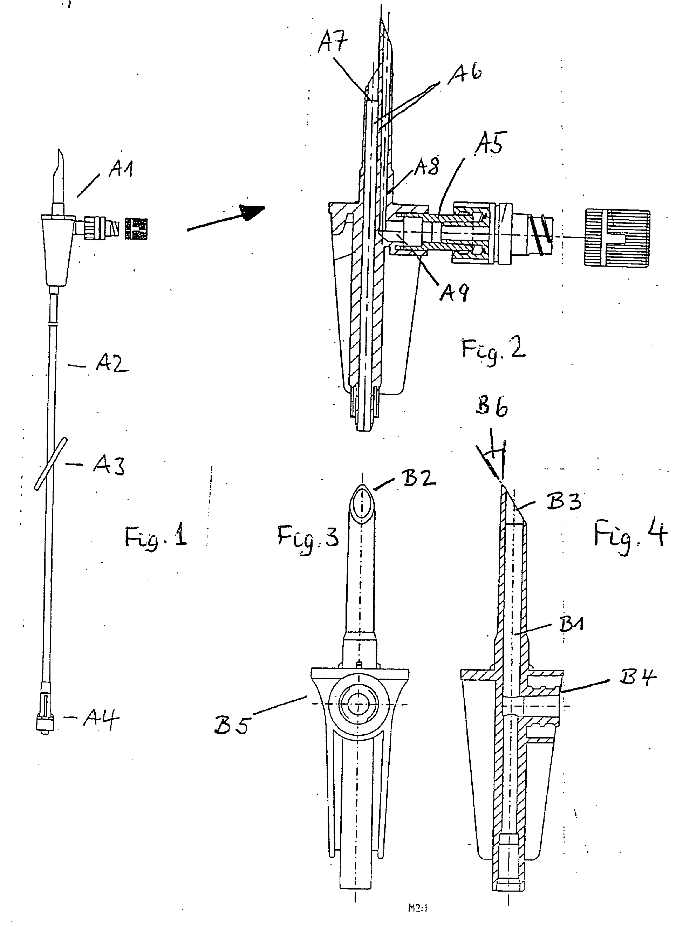

Es wird Bezug genommen auf den in den Figuren 1 und 2 gezeigten Stand der Technik.

Häufig werden in der Infusionstherapie Medikamente über separate Übertragungssets verabreicht, die an ein Hauptinfusionssystem gekoppelt werden. Viele dieser Medikamente müssen vor der Verabreichung gemischt bzw. aufgelöst werden, da sie wegen der besseren Haltbarkeit in gefriertrockener Pulverform in den Medikamentenflaschen abgepackt sind. Das vorbekannte Set wurde u.a. auch für die Zubereitung von Zytostatika mit Infusionsbeuteln entwickelt, wobei auf Kanülen wegen der Verletzungs- und Kontaminationsgefahr verzichtet wurde, ebenso wie die Zubereitung und die Übertragung der Medikamente leckagen- und kontaminationsfrei war. Über einen speziellen Luer-Lock negativ Anschluß A1 mit einem Verschlußmechanismus konnte Flüssigkeit mit einer Spritze aus dem Beutel genommen werden. Die Trägerlösung wurde dann mit dem gefriergetrocknetem Medikament unter der Verwendung eines Entnahme-Zugabespikes gemischt und anschließend in den Infusionsbeutel zurückgegeben. Nach dem Entfernen der Spritze war das Set fertig zum Anschluß an das Hauptverabreichungsset, der einen Übertragungsschlauch A2 mit Klemme A3 und einem Luer-Lock positiv Anschluß A4 besaß (siehe Fig.1).

Der Flüssigkeitskanal A6 war doppellumig und besaß einerseits einen Durchmesser von 1,9 mm bzw. 1,1 mm bzw. einen Querschnitt von 0,95 mm² (A7 bzw. A8, siehe Fig.2). Das Totvolumen A9 im Bereich des Anschlußstutzens betrug 0,28 cm³.

Die Figuren 3 bis 5 hingegen zeigen den Aufbau des erfindungsgemäßen Einstechdorns ohne bzw. mit aufgeschraubten Adapter. Deutlich ist zu sehen, daß bei gleichen Größenverhältnissen der Querschnitt des durchgehenden Flüssigkeitskanals B1 größer ist als beim Stand der Technik. Letzteres trifft sowohl für den oberen, als auch für den unteren Bereich zu. Erfindungsgemäß liegt er im Bereich von 2,5 bis 6 mm, insbesondere 3,4 mm (oberer Bereich) bzw. 2,6 mm (unterer Bereich), was eine prozentuale Vergrößerung von mindestens > 30 % darstellt. Hierdurch wird verständlicherweise ein größerer Flüssigkeitsdurchsatz pro Zeiteinheit erreicht, was in den meisten Fällen wünschenswert ist. Die Handhabung wird dadurch leichter und schneller.

Gegenüber der extrem spitz zulaufenden Spitze ist die Spitze B2 des erfindungsgemäßen Einstechdorns abgerundet und besitzt eine große ovale Öffnung mit einer relativ dünnen Wandung, wodurch das Risiko der Wandperforation eines Innenbeutels (nicht gezeigt) vermieden wird. Die große ovale Einlauföffnung besitzt einen Querschnitt von 0,18 cm² und verhindert eine Blockade des Flüssigkeitstransfers beim etwaigen Kontakt mit der Beutelwandung. Insbesondere ist daran gedacht, eine Spitze im Winkelbereich B6 von 25° bis 35°, insbesondere 30° zur Senkrechten vorzusehen. Aus Fig. 3 ist ferner gut ersichtlich, daß die Griffplatte in ihrem oberen Bereich B5 eine abgerundete Konfiguration besitzt, wodurch wiederum die leichte und schnelle Handhabung des Einstechdorns begünstigt wird. Aus Fig. 4 ist in der Seitenansicht des erfindungsgemäßen Einstechdorns gut zu sehen, daß ein Luer-Lock negativ Anschluß B4 im Anschlußstutzen integriert ist, der die direkte Montage/Verklebung von Anschlußstücken mit Absperrmechanismen oder Zuspritzmembranen (nicht gezeigt) an den Einstechdorn ermöglicht. Hierdurch wird die Verwendung eines zusätzlichen Verbindungsstückes vermieden, das gemäß Fig. 2 erforderlich war und mit A5 dort gekennzeichnet ist. Es dürfte einleuchten, daß hierdurch die Herstellung vereinfacht und die Wirtschaftlichkeit erhöht wird. Außerdem wird in grundsätzlicher Hinsicht die Sicherheit des erfindungsgemäßen Einstechdorns durch weniger Montage/Klebestellen erhöht. In dem angesprochenen Bereich wird auch das Totraumvolumen B7 (von ca. 0,28 cm³ auf etwa 0,15 cm³ und geringer) reduziert.

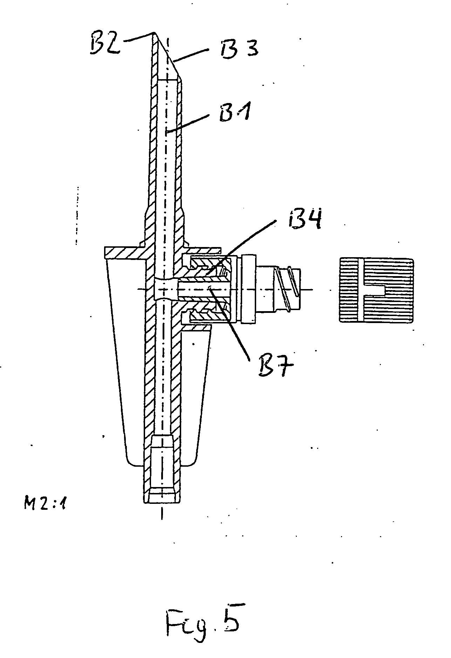

Aus Fig. 5 ist schließlich ersichtlich, daß ein bekannter Adapter C1 an den Luer-Lock negativ Anschluß B4 angeschlossen werden kann.

Die gezeigten Größenverhältnisse der Figuren 2 bis 5 sind im Maßstab 2:1 gezeigt. Es dürfte jedoch einleuchten, daß andere Größenverhältnisse ebenfalls denkbar sind. Der Erfindungsgemäße Einstechdorn besteht aus ABS. Andere geeignete Materialien sind für den Fachmann jedoch denkbar.

## Patentansprüche

1. Einstechdorn für ein Medikamentenmisch- und Verabreichungsset der Infusionstherapie zum Entnehmen von Flüssigkeit aus einem Behältnis mit einem durchgehenden einlumigen Flüssigkeitskanal zwischer einem ersten Anschluss und einer Spitze mit abgerundeter (B2) und über den Gesamtquerschnitt des Flüssigkeitskanals (B1) schrägverlaufender Form (B3), einer Griffplatte und einem vom Flüssigkeitskanal abzweigenden zweiten Anschluss in Form eines Anschlußstutzens (B4) wobei der Einstechdorn einteilig ausgebildet ist, und
a. der Flüssigkeitskanal (B1) einen Innendurchmesser im Bereich von 2,5 bis 3,5 mm aufweist,
b. der Anschlußstutzen in Form eines Luer-(Lock) negativ oder zylindrischen Anschluß (B4) ausgebildet ist,
c. das Volumen des vom Flüssigkeitskanal abzweigenden Anschlußstutzens (Totraumvolumen B7) höchstens 0,15 cm³ beträgt.

2. Einstechdorn nach Anspruch 1, **dadurch gekennzeichnet, daß** er aus Polycarbonat, Polyamid, Polystyrol oder Acrylnitril-butadienstyrol besteht.

3. Einstechdorn nach Anspruch 1 und 2, **dadurch gekennzeichnet, daß** die schrägverlaufende Form der Spitze einen Winkel (B6) zum Flüssigkeitskanal (B1) im Bereich von 25° bis 35° besitzt.

4. Einstechdorn nach Anspruch 1 bis 3, **dadurch gekennzeichnet, daß** die Griffplatte im oberen Bereich (B5) eine abgerundete Kontur aufweist.

5. Einstechdorn nach Anspruch 1 bis 5, **gekennzeichnet durch** eine ovale Öffnung an der Spitze des Flüssigkeitskanals (B1).

## Claims

1. Puncturing mandrel for a medicament mixing and administration set in infusion therapy for removing liquid from a container with a through, single-opening liquid duct between a first connection and a tip with a rounded (B2) shape (B3) sloping over the total cross-section of the liquid duct (B1), a holding plate and a second connection in the form of a connecting piece (B4) branching off from the liquid duct, the puncturing mandrel being constructed in one piece and
a. the liquid duct (B1) has an internal diameter in the range 2.5 to 3.5 mm,
b. the connecting piece in the form of a Luer lock has a negative or cylindrical connection (B4)
c. the volume of the connecting piece branching off from the liquid duct (clearance volume B7) is max. 0.15 cm³.

2. Puncturing mandrel according to claim 1, **characterized in that** it is made from polycarbonate, polyamide, polystyrene or acrylonitrile-butadiene styrene.

3. Puncturing mandrel according to claim 1 and 2, **characterized in that** the sloping shape of the tip has an angle (B6) to the liquid duct (B1) in the range 25 to 35°.

4. Puncturing mandrel according to claims 1 to 3, **characterized in that**, in the upper area (B5), the holding plate has a rounded contour.

5. Puncturing mandrel according to claims 1 to 5, **characterized by** an oval opening at the tip of the liquid duct (B1).

## Revendications

1. Mandrin de percée pour un kit de mélange et d'administration de médicaments de la thérapie d'infusion destiné à prélever du liquide d'un récipient, comportant un canal de liquide traversant à un lumen entre un premier raccord et une pointe de forme arrondie (B2) et s'étendant obliquement (B3) sur l'ensemble de la section transversale du canal de liquide (B1), une plaque de préhension et un deuxième raccord sous la forme d'une tubulure de raccordement (B4) en ramification du canal de liquide, le mandrin de percée étant réalisé d'un seul tenant, et
a. le canal de liquide (B1) ayant un diamètre intérieur se situant dans la plage de 2,5 à 3,5 mm,
b. la tubulure de raccordement étant réalisée sous la forme d'un raccord Luer-(Lock) (B4) négatif ou cylindrique,
c. le volume de la tubulure de raccordement (volume d'espace mort B7) dérivant du canal de liquide étant au maximum de 0,15 cm³.

2. Mandrin de percée selon la revendication 1, **caractérisé en ce qu'**il est en polycarbonate, en polyamide, en polystyrène ou en acrylonitrile-butadiènestyrène.

3. Mandrin de percée selon la revendication 1 ou 2, **caractérisé en ce que** la forme s'étendant obliquement de la pointe forme avec le canal de liquide (B1) un angle (B6) de l'ordre de 25° à 35°.

4. Mandrin de percée selon l'une des revendications 1 à 3, **caractérisé en ce que** le contour de la plaque de préhension est arrondi dans la zone supérieure (B5).

5. Mandrin de percée selon l'une des revendications 1 à 5, **caractérisé par** un orifice ovale à la pointe du canal de liquide (B1).
